# EUROPEAN PATENT APPLICATION

(11) **EP 3 269 807 A1**
(43) Date of publication of application: **17.01.2018**
(21) Application number: 15884707.9
(22) Date of filing: 18.12.2015
(51) Int. Cl.: C12N 11/00, C12N 11/14

(54) **IMMOBILIZED PROTEASE WITH IMPROVED RESISTANCE TO CHANGE IN EXTERNAL ENVIRONMENT**

(30) Priority: 09.03.2015 JP 2015046380
(71) Applicant: Shimadzu Corporation, Kyoto-shi, Kyoto 604-8511 (JP)
(72) Inventor: SHIMADA, Takashi, Kyoto-shi Kyoto 604-8511 (JP); IWAMOTO, Noriko, Kyoto-shi Kyoto 604-8511 (JP)
(74) Representative: Webster, Jeremy Mark
(86) International application number: PCT/JP2015/085444
(87) International publication number: WO 2016/143223

(57) **Abstract**

The present invention provides a highly active protease that can be used in sample preparation for mass spectrometry of a protein and has excellent stability against a change in an external environment.

The present invention provides an immobilized protease characterized in that a crudely purified protease or a protease that has not been subjected to a self-digestion resistance treatment is immobilized on surfaces of nanoparticles, and provides a method for producing the immobilized protease. The immobilized protease of the present invention can maintain high activity without being subjected to a change in an external environment, and thus is effective, for example, in preparing a sample to be supplied for mass spectrometry of a protein in a clinical specimen.

## Description

### [Technical Field]

The present invention relates to an immobilized protease that has an improved resistance to a change in an external environment and can be used for sample preparation for mass spectrometry of a protein.

### [Technical Background]

Along with progress in mass spectrometry technologies, development of a high-throughput analysis platform for multi-specimen processing for proteins has also been advanced. However, protein analysis, which is a foundation for the development, is behind genome analysis. The reason is that, in genome analysis, there are many very general and quantitative techniques for sample preparation, such as PCR and plasmid amplification, whereas in protein analysis, a technique for preparing samples with high reproducibility has not been established. Firstly, for proteins, an efficient amplification technique has not yet been established. Secondly, when a target of mass spectrometry is a large protein such as an antibody, it is difficult to analyze the protein as it is and thus, as a pretreatment, the protein is digested and fragmented using a protease. However, reproducibility of the digestion reaction is not sufficiently ensured.

As an attempt to increase reproducibility of a digestion reaction of a protein, binding of a protease to a solid phase carrier has been performed. So far, there are numerous reports that enzyme activity and a reaction speed are improved by binding a protease such as trypsin to a solid phase carrier, such as a glass surface, a membrane, a hollow fiber, a polymer, a gel, a sol, or porous silica (Non-Patent Documents 1 - 5 and the like). Further, it has been reported that nanoparticles on which trypsin is immobilized are used in a method in which a protein is selectively hydrolyzed by limiting access to a substrate of a protease (Non-Patent Document 6). Performance of these immobilized proteases is evaluated based on indicators such as whether or not an immobilized protease can be repeatedly used, whether or not an immobilized protease can be used in digestion reaction in a short time, and whether or not a peptide sequence recovery rate (sequence coverage) of a substrate protein is sufficient. However, all the reports so far are only qualitative evaluations, not evaluations based on quantitative physicochemical properties. Further, no consideration has been given on stability against a change in an external environment such as temperature, pH, preparation of an immobilized protease, and various reagents used in reaction with a substrate protein.

### [Related Art]

### [Non-Patent Documents]

[Non-Patent Document 1] Junfeng Ma, at. al., Organic-Inorganic Hybrid Silica Monolith Based Immobilized Trypsin Reactor with High Enzymatic Activity, Analytical Chemistry, 2008, 80, 2949.
[Non-Patent Document 2] J.Robert Freije, at. al., Chemically Modified, Immobilized Trypsin Reactor with Improved Digestion Efficiency, Journal of Proteome Research, 2005, 4, 1805.
[Non-Patent Document 3] Maria T.Dulay, at. al., Enhanced Proteolytic Activity of Covalently Bound Enzymes in Photopolymerized Sol Gel, Analytical Chemistry, 2005, 77, 4604.
[Non-Patent Document 4] Jana Krenkova, at. al., Highly Efficient Enzyme Reactors Containing Trypsin and Endoproteinase LysC Immobilized on Porous Polymer Monolith Coupled to MS Suitable for Analysis of Antibodies, Analytical Chemistry, 2009, 81, 2004.
[Non-Patent Document 5] Yan Li, at. al., Immobilization of Trypsin on Superparamagnetic Nanoparticles for Rapid and Effective Proteolysis, Journal of Proteome Research, 2007, 6, 3849.
[Non-Patent Document 6] Noriko Iwamoto at. al., Selective detection of complementarity determining regions of monoclonal antibody by limiting protease access to the substrate: nanosurface and molecular-orientation limited proteolysis, Analyst, 2014, 139, 576.

### [Summary of the Invention]

### [Problems to Be Solved by the Invention]

The present invention is intended to provide a highly active protease that can be used in sample preparation for mass spectrometry of a protein and has excellent stability against a change in an external environment.

### [Means for Solving the Problems]

As a result of intensive studies to solve the above problems,the present inventors found that, by immobilizing a protease such as trypsin on nanoparticles, even for a low purity protease that has not been subjected to functional enhancement such as reductive dimethylation, high activity can be maintained over wide ranges of temperatures and pH values, and the protease is not affected by an organic solvent or a surfactant, and thus accomplished the present invention.

That is, the present invention includes the following aspects.
(1) An immobilized protease is obtained by immobilizing a crudely purified protease or a protease that has not been subjected to a self-digestion resistance treatment on surfaces of nanoparticles.
(2) In the immobilized protease described in the above aspect (1), the nanoparticle has a particle size of 100 - 500 nm.
(3) In the immobilized protease described in any one of the above aspects (1) and (2), the nanoparticle is a magnetic nanoparticle.
(4) In the immobilized protease described in any one of the above aspects (1) - (3), the protease is trypsin, chymotrypsin, lysyl endopeptidase, V8 protease, Asp N protease, Arg C protease, papain, pepsin, or dipeptidyl peptidase.
(5) In the immobilized protease described in any one of the above aspects (1) - (3), the self-digestion resistance treatment is a reductive dimethylation treatment.
(6) In the immobilized protease described in the above aspect (5), the protease is trypsin or lysyl endopeptidase.
(7) A method for preparing an immobilized protease includes a process of immobilizing a crudely purified protease or a protease that has not been subjected to a self-digestion resistance treatment on surfaces of nanoparticles.
(8) A method of imparting, to a crudely purified protease or a protease that has not been subjected to a self-digestion resistance treatment, resistance to a change in an external environment by immobilizing the protease on surfaces of nanoparticles.
(9) In the method described in any one of the above aspects (7) and (8), the protease is trypsin, chymotrypsin, lysyl endopeptidase, V8 protease, Asp N protease, Arg C protease, papain, pepsin, or dipeptidyl peptidase.
(10) In the method described in any one of the above aspects (7) and (8), the self-digestion resistance treatment is a reductive dimethylation treatment.
(11) In the method described in the above aspect (10), the protease is trypsin or lysyl endopeptidase.

The present application claims the priority of Japanese Patent Application No. 2015-046380 filed on March 9, 2015 and includes the content described in the specification of the patent application.

### [Effect of the Invention]

The immobilized protease of the present invention obtained by immobilizing a protease on surfaces of nanoparticles can maintain high activity without being affected by a change in an external environment such as temperature, pH, and additives, and has excellent stability. Therefore, the immobilized protease of the present invention can improve reliability and reproducibility of data obtained using mass spectrometry by using the immobilized protease for sample preparation of a peptide fragment for quantification or identification of a protein using a mass spectrometry method. The immobilized protease of the present invention can achieve excellent performance of a mass spectrometry grade regardless of a type and purity of the protease immobilized on a nanoparticle. Therefore, it is economical to use the immobilized protease of the present invention as a bundled reagent of a kit for quantification or identification of a protein using mass spectrometry, and can improve profitability of the reagent.

### [Brief Description of the Drawings]

[Fig. 1] Fig. 1 shows enzyme activities of trypsin at various pH values (pH 6.5, pH 7.0, pH 7.5, pH 8.0, pH 8.5, pH 9.0) in the presence of various additives (urea, OTG, MeCN) at 25 °C (upper panel: immobilized trypsin (FG-Gold, FG-TPCK); lower panel: non-immobilized trypsin (Gold, TPCK)).
[Fig. 2] Fig. 2 shows enzyme activities of trypsin at various pH values (pH 6.5, pH 7.0, pH 7.5, pH 8.0, pH 8.5, pH 9.0) in the presence of various additives (urea, OTG, MeCN) at 37 °C (upper panel: immobilized trypsin (FG-Gold, FG-TPCK); lower panel: non-immobilized trypsin (Gold, TPCK)).
[Fig. 3] Fig. 3 shows enzyme activities of trypsin at various pH values (pH 6.5, pH 7.0, pH 7.5, pH 8.0, pH 8.5, pH 9.0) in the presence of various additives (urea, OTG, MeCN) at 45 °C (upper panel: immobilized trypsin (FG-Gold, FG-TPCK); lower panel: non-immobilized trypsin (Gold, TPCK)).
[Fig. 4] Fig. 4 shows enzyme activities of trypsin at various pH values (pH 6.5, pH 7.0, pH 7.5, pH 8.0, pH 8.5, pH 9.0) in the presence of various additives (urea, OTG, MeCN) at 50 °C (upper panel: immobilized trypsin (FG-Gold, FG-TPCK); lower panel: non-immobilized trypsin (Gold, TPCK)).
[Fig. 5] Fig. 5 shows enzyme activities of trypsin at various pH values (pH 6.5, pH 7.0, pH 7.5, pH 8.0, pH 8.5, pH 9.0) in the presence of various additives (urea, OTG, MeCN) at 60 °C (upper panel: immobilized trypsin (FG-Gold, FG-TPCK); lower panel: non-immobilized trypsin (Gold, TPCK)).
[Fig. 6] Fig. 6 shows enzyme activities of trypsin at various pH values (pH 6.5, pH 7.0, pH 7.5, pH 8.0, pH 8.5, pH 9.0) in the presence of various additives (urea, OTG, MeCN) at 70 °C (upper panel: immobilized trypsin (FG-Gold, FG-TPCK); lower panel: non-immobilized trypsin (Gold, TPCK)).
[Fig. 7] Fig. 7 shows enzyme activities of trypsin at pH 8.0 in the presence of various additives (DTT, TCEP, CHAPS, SDS, Tween 20, Triton X-100, NP-40) at 37 °C (upper panel: immobilized trypsin (FG-Gold, FG-TPCK); lower panel: non-immobilized trypsin (Gold, TPCK)).
[Fig. 8] Fig. 8 shows enzyme activities of trypsin at pH 8.0 in the presence of various additives (NaCl, AS, IAA, Trehalose, Glycerol, EDTA) at 37 °C (upper panel: immobilized trypsin (FG-Gold, FG-TPCK); lower panel: non-immobilized trypsin (Gold, TPCK)).
[Fig. 9] Fig. 9 shows enzyme activities of trypsin at various pH values (pH 6.5, pH 7.0, pH 7.5, pH 8.0, pH 8.5, pH 9.0) at 37 °C (FG-Gold, FG-TPCK: nanoparticle immobilized trypsin; CR-TPCK, AR-TPCK: microparticle immobilized trypsin).
[Fig. 10] Fig. 10 shows enzyme activities of trypsin at pH 8.0 in the presence of various additives (urea, NaCl, AS, IAA, EDTA) at 37 °C (FG-Gold, FG-TPCK: nanoparticle immobilized trypsin; CR-TPCK, AR-TPCK: microparticle immobilized trypsin).
[Fig. 11] Fig. 11 shows enzyme activities of trypsin at pH 8.0 in the presence of various additives (Trehalose, Glycerol) at 37 °C (FG-Gold, FG-TPCK: nanoparticle immobilized trypsin; CR-TPCK, AR-TPCK: microparticle immobilized trypsin).

### [Mode for Carrying Out the Invention]

In the following, the present invention is described in detail.

The immobilized protease of the present invention is characterized in that nanoparticles are used as a solid phase carrier and the protease is immobilized on surfaces of the nanoparticles. The immobilized protease of the present invention can maintain high activity without being affected by a change in an external environment even for a crudely purified protease or a protease that has not been subjected to a self-digestion resistance treatment by immobilizing the protease on a nanoparticle.

A size of a nanoparticle used in the present invention is not limited as long as a protease can be bound to a surface of the nanoparticle at multiple points. However, since a protease such as trypsin or lysyl endopeptidase has a molecular diameter of about 5 nm, the particle size of the nanoparticle is preferably 100 - 500 nm, more preferably 150 - 400 nm, and even more preferably 200 - 300 nm. When the particle size is increased (for example, to an order of a micrometer), it is necessary to consider shrinkage of particles due to influence of an additive and the like. However, in the case of a nanoparticle and immobilizing a protease on the surface of the nanoparticle, it is not necessary to consider this contraction and thus it is possible to prepare a more stable enzyme. Here, the "particle size" refers to a particle size having a highest appearance frequency in a particle distribution, that is, a central particle size.

An amount of a protease to be immobilized with respect to nanoparticles varies depending on the particle size of the nanoparticles, and the kind and purity of the protease, but is generally 1 - 10% by weight, and preferably 2 - 5% by weight with respect to 1% by weight of the nanoparticles.

As a kind of the nanoparticles, magnetic nanoparticles that can be dispersed or suspended in an aqueous medium and can be easily recovered from the dispersion or suspension by magnetic separation or magnetic precipitation separation are preferable. Further, from a point of view that aggregation is less likely to occur, magnetic nanoparticles covered with an organic polymer are more preferable. Examples of base materials of magnetic nanoparticles include ferromagnetic alloys such as iron oxide (magnetite (Fe₃O₄), maghemite (γ-Fe₂O₃)), and ferrite (Fe/M)₃O₄. In the ferrite (Fe/M)₃O₄, M means a metal ion that can be used together with an iron ion to form a magnetic metal oxide, and typically, Co²⁺, Ni²⁺, Mn²⁺, Mg²⁺, Cu²⁺, Ni²⁺ and the like are used. Further, examples of the organic polymer covering the magnetic nanoparticles include polyglycidyl methacrylate (poly GMA), a copolymer of GMA and styrene, polymethyl methacrylate (PMMA), polymethyl acrylate) (PMA), and the like. Specific examples of magnetic nanoparticles coated with an organic polymer include FG beads, SG beads, Adembeads, nanomag, and the like. As a commercially available product, for example, FG beads (polymer magnetic nanoparticles having a particle size of about 200 nm obtained by coating ferrite particles with polyglycidyl methacrylate (poly GMA)) manufactured by Tamagawa Seiki Co., Ltd. is suitably used.

In order to suppress adsorption of a nonspecific protein and to selectively immobilize a protease, it is preferable that the nanoparticles be modified with spacer molecules capable of binding to the protease. By immobilizing a protease via a spacer molecule, desorption of the protease from surfaces of nanoparticles is suppressed, and position selectivity of protease digestion is improved.

Further, by adjusting a molecular size of a spacer, a protease can be caused to selectively access a desired position of a substrate protein, and position selectivity can be improved.

A spacer preferably can bind to protease and does not inactivate a protease. From a point of view of controlling an access range of a protease immobilized on surfaces of nanoparticles, a spacer preferably has a small molecular diameter. The molecular diameter of the spacer is preferably 5 nm or less, more preferably 3 nm or less, and even more preferably 2 nm or less. Further, a molecular weight of the spacer is preferably 2000 or less, more preferably 1500 or less, and even more preferably 1000 or less.

A spacer molecule having the above molecular diameter and capable of immobilizing a protease is preferably a non-protein, and is preferably a molecule having a functional group at a terminal, examples of the functional group including an amino group, a carboxyl group, an ester group, an epoxy group, a tosyl group, a hydroxyl group, a thiol group, an aldehyde group, a maleimide group, a succinimide group, an azide group, a biotin, an avidin, and a chelate. For example, for immobilization of trypsin, a spacer molecule having an activated ester group is preferred. Further, of a spacer molecule, as a spacer arm portion other the functional group, a hydrophilic molecule can be used, examples of the hydrophilic molecule including polyethylene glycol and its derivatives, polypropylene glycol and its derivatives, polyacrylamide and its derivatives, polyethyleneimine and its derivatives, poly (ethylene oxide) and its derivatives, poly (ethylene terephthalic acid) and its derivatives, and the like.

Nanoparticles surface-modified with such spacer molecules are also commercially available, and these nanoparticles can be used. For example, nanoparticles modified with a spacer molecule having an ester group (active ester group) activated with N-hydroxysuccinimide is commercially available under a trade name "FG beads NHS" (Tamagawa Seiki Co., Ltd.).

A method for immobilizing a protease on surfaces of nanoparticles is not particularly limited. An appropriate method can be adopted according to characteristics of the protease and the nanoparticles (or spacer molecules modifying the surfaces of the nanoparticles). However, an amine coupling method of the nanoparticles and the protease via the functional groups of the spacer molecules is preferable. For example, a carboxyl group surface-modified on nanoparticles can be esterified with N-hydroxysuccinimide (NHS) to form an activated ester group to which an amino group of a protease can be bound. This coupling reaction can be performed in the presence of carbodiimide as a condensing agent, examples of the carbodiimide including 1-ethyl-3- (3-dimethylaminopropyl) carbodiimide (EDAC), N,N'-dicyclohexylcarbodiimide (DCC), bis(2,6-diisopropylphenyl) carbodiimide (DIPC), and the like. Further, an amino group of a protease may be bound to an amino group surface-modified on nanoparticles using a cross-linking agent such as glutaraldehyde, bifunctional succinimide, bis(sulfosuccinimidyl) suberate (BS3), sulfonyl chloride, maleimide, and pyridyl disulfide.

The coupling method of the nanoparticles and the protease via the functional groups of the spacer molecules can be performed by a simple operation of adding a protease solution to a suspension of the nanoparticles and mixing and stirring the mixture under certain conditions. The reaction conditions are not particularly limited. However, for example, the suspension of the nanoparticles, to which the protease is added, is stirred at a temperature of 1 - 10 °C at pH 7.0 at 50 - 200 rpm for 0.5 - 1 hour.

After the protease is immobilized on the surfaces of the nanoparticles, it is preferable to inactivate an active portion that is not bound to the protease on the surfaces of the nanoparticles. For example, when spacer molecules on which the protease is not immobilized exist on the surfaces of the nanoparticles, problems may occur such as that the unbound spacer molecules bind to contaminants in the sample and adversely affects protease digestion, and that peptide fragments produced by protease digestion are immobilized on the nanoparticles. After the protease is immobilized, by blocking unbound spacer molecules, such problems are suppressed. As a method for inactivating the active portion unbound to the protease, chemical modification is preferred. For example, an activated ester group can be inactivated by reacting with a primary amine to form an amide bond.

In the present invention, a kind of a protease to be immobilized on nanoparticles may be appropriately selected according to a kind of a protein to be quantified or identified using mass spectrometry, and is not limited. Examples of the protease include crudely purified proteases or proteases that have not been subjected to a self-digestion resistance treatment, such as trypsin (peptide is cleaved at a C-terminal side of basic amino acid residues (Arg and Lys)), chymotrypsin (peptide is cleaved at a C-terminal side of aromatic amino acid residues (Phe, Tyr and Trp)), lysyl endopeptidase (peptide is cleaved at a C-terminal side of a Lys residue), V8 protease (peptide is cleaved at a C-terminal side of a Glu residue), Asp N protease (peptide is cleaved at an N-terminal side of an Asp residue), Arg C protease (peptide is cleaved at a C-terminal side of an Arg residue), papain, pepsin, and dipeptidyl peptidase. Among the above proteases, trypsin is particularly preferably used in the present invention. Trypsin has a small molecular diameter, and an active site exists inside a molecule. Therefore, a region where the active site can access a substrate protein is restricted, and position selectivity of protease digestion can be improved. In particular, when the substrate protein is an antibody, it is preferable to use trypsin as the protease.

The protease used in the present invention is a crudely purified or a protease that has not been subjected to a self-digestion resistance treatment, and purity of the protease is not limited. Therefore, when a commercially available protease is used, the protease is not limited to a protease of a mass spectrometry grade or a protease of a sequencing (sequence) grade, and may be a native protease derived from a living body. For example, in the case of trypsin, native trypsin derived from a living body generates pseudo trypsin showing chymotrypsin-like activity by self-digestion. Therefore, trypsin having reduced chymotrypsin activity by being subjected to an N-tosyl-L-phenylalanine chloromethylketone (TPCK) treatment, or, trypsin having increased resistance to self-digestion by subjecting a lysine residue thereof to a reductive dimethylation treatment, is commercially available as trypsin of a mass spectrometry grade. However, the trypsin used in the present invention may be trypsin for which such a reductive dimethylation treatment of a lysine residue is not performed.

Protease bound to nanoparticles as described above has dramatically improved resistance to a change in an external environment. Here, the term "external environment" refers to temperature (heat), pH, an organic solvent, a protein denaturing agent, a protein reducing and alkylating agent, a protein protecting and stabilizing agent, a surfactant for solubilizing a protein, a salting-out agent, salts, and the like. Examples of protein denaturing agents include urea, guanidine hydrochloride, dithiothreitol (DTT), mercaptoethanol, and the like. Examples of protein reducing and alkylating agents include tris (2-carboxyethyl) phosphine hydrochloride (TCEP), iodoacetamide (IAA), and the like. Examples of protein protecting and stabilizing agents include chelating agents such as EDTA, polyols such as glycerol, sugars such as trehalose, glucose and sucrose, and the like. Examples of organic solvents include acetonitrile, methanol, ethanol, isopropanol, and the like. Examples of the surfactant include polyoxyethylene nonionic surfactant (such as Triton X-100, Tween 20/40/60/80, and Nonidet P-40 (NP-40)), alkyl glycoside-based nonionic surfactant (such as n-octyl-β-D-glucoside (OG), n-octyl-β-D-thioglucoside (OTG), n-dodecyl- β-D-maltoside (DDM), and n-nonyl-β-D-maltoside (NG)), amphoteric surfactant (such as 3-[(3-cholamidopropyl) dimethylammonio]-1-propanesulfonic acid (CHAPS) or 3-[(3-cholamidopropyl) dimethylammonio]-2-hydroxypropanesulfonic acid (CHAPSO)), and cationic surfactant (such as cetyltrimethylammonium bromide (CTAB)). Examples of salting-out agents include ammonium sulfate (AS). Examples of salts include sodium chloride, potassium chloride, sodium acetate, magnesium sulfate, and the like.

Conditions in the case in which a substrate protein is digested using the immobilized protease of the present invention are not particularly limited, and conditions similar to general protease digestion can be suitably adopted. For example, it is preferable to incubate at a temperature of about 37 °C for about 1 hour - 20 hours in a buffer solution adjusted to a vicinity of an optimum pH of the protease. Further, a quantitative mixing ratio of a substrate protein to an immobilized protease is not particularly limited, and may be set so as to have an amount of the protease corresponding to an amount of the substrate protein. A general protease digestion condition is that the ratio (substrate protein):(protease) is about 100:1 - 10:1 (weight ratio).

Mass spectrometry is suitable for identification and quantification of a substrate protein from a peptide fragment produced by digestion of the substrate protein using the immobilized protease of the present invention Mass spectrometry can determine an amino acid sequence and thus can determine whether or not a peptide fragment is a peptide fragment derived from a specific protein such as an antibody. Further, based on a peak intensity, concentration of peptide fragments in a sample can be determined. An ionization method in mass spectrometry is not particularly limited, and an electron ionization (EI) method, a chemical ionization (CI) method, a field desorption (FD) method, a fast atom collision (FAB) method, a matrix assisted laser desorption ionization (MALDI) method, an electrospray ionization (ESI) method, and the like can be adopted. A method for analyzing an ionized sample is also not particularly limited, and a method of a magnetic field deflection type, a quadrupole (Q) type, an ion trap (IT) type, a time of flight (TOF) type, a Fourier transform ion cyclotron resonance (FT-ICR) type, or the like can be appropriately determined according to the ionization method. Further, MS/MS analysis or multistage mass spectrometry of MS3 or higher can also be performed using triple quadrupole mass spectrometer or the like.

The immobilized protease of the present invention can stably maintain high activity in a state of being immobilized on surfaces of nanoparticles and thus can be provided as a component of a kit for sample preparation of a peptide fragment for quantification or identification of a protein using a mass spectrometry method. The immobilized protease of the present invention is particularly suitable for detecting and quantifying an antibody. By selectively protease-digesting an Fab region and subjecting an obtained peptide fragment sample to mass spectrometry, a sequence and an amount of a peptide fragment containing an amino acid sequence of a complementarity determining region can be determined. The immobilized protease of the present invention can also be used in analysis of pharmacokinetics, analysis of interaction using an antigen antibody reaction, various interactome analysis, basic research such as identification of immunoprecipitated protein, sequence analysis of biomolecule drugs such as antibody drugs, quality assurance, identity check test for generic drugs, and the like.

In the following, the present invention is described in detail based on Examples. However, the present invention is not limited by these Examples.

For reagents used in the following Examples, those not specifically described were obtained from Wako Pure Chemical Industries. Further, pH values of the following buffers were adjusted using a precision pH meter.
HEPES buffer: 25 mM HEPES-NaOH, pH 7.0
Ethanolamine buffer: 1 M ethanolamine-HCl, pH 8.0
Tris buffer: 25 mM Tris-HCl, pH 8.0

### (Example 1) Preparation of Immobilized Protease

As nanoparticles for protease immobilization, FG beads (FG beads NHS manufactured by Tamagawa Seiki) having an average particle size of 190 nm (dispersion range: ± 20 nm) modified with a spacer (see the following chemical formula (where L is a binding site to a surface of a nanoparticle); spacer length: 1 nm) of which a carboxy group was activated with N-hydroxysuccinimide were used.

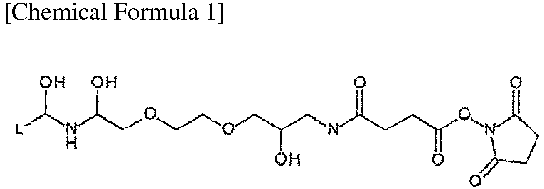

50 µl of an isopropanol suspension of 1 mg of FG beads was centrifuged at 4 °C (15000 rpm, 5 minutes) to precipitate the nanoparticles, and supernatant was removed, and thereafter, washing with methanol was performed. A solution obtained by dissolving a solution containing 50 µg of a protease in 200 µL of a HEPES buffer was added to the nanoparticles to suspend the nanoparticles. In forming the suspension, an ultrasonic treatment was performed for a few seconds so that a temperature of the suspension did not rise.

The suspension of the nanoparticles was stirred at 4 °C for 30 minutes and then centrifuged (15000 rpm, 5 minutes) at 4 °C to precipitate the nanoparticles, and supernatant was removed. Subsequently, 200 µL of an ethanolamine buffer was added to suspend the particles, and the mixture was stirred at 4 °C for 30 minutes, and excess N-hydroxysuccinimide groups on the surfaces of the nanoparticles were blocked with ethanolamine, and a nanoparticle-immobilized protease (50 µg/mg, solid phase) was obtained. Thereafter, centrifugation (15000 rpm, 5 minutes) at 4 °C was performed to precipitate the nanoparticles, and supernatant was removed. Thereafter, washing with a Tris buffer and centrifugation were repeated twice, and a suspension in a Tris buffer (100 µL) was formed (protease concentration in the suspension: 0.5 µg/µL).

### (Example 2) Enzyme Stability Test 1 (Comparison between Nanoparticle-Immobilized Protease and Non-Immobilized Protease)

Enzyme stability was examined by performing enzymatic reactions under various conditions by using, as a protease substrate, N-α-benzoyl-DL-arginine-p-nitroanilide hydrochloride (MW = 434.9), and using, as proteases, two kinds of trypsins including Trypsin Gold, Mass Spec Grade (manufactured by Promega) (hereinafter referred to as "Gold") and Trypsin TPCK Treated from bovine pancreas, Product Number T1426 (manufactured by Sigma Aldrich) (hereinafter referred to as "TPCK"), and "FG-Gold" and "FG-TPCK" that are respectively obtained by immobilizing "Gold" and "TPCK" on nanoparticles according to the method described in Example 1. "Gold" is a protease of a mass spectrometry grade that, by performing a reductive dimethylation treatment in addition to a chymotrypsin inactivation treatment (TPCK treatment), is resistant to self-digestion and broadly maintains high activity without depending on temperature and pH. On the other hand, "TPCK" is a protease for which, although a chymotrypsin inactivation treatment is performed, due to a low degree of purification, chymotrypsin derived from impurities remains and chymotrypsin activity is not completely suppressed, and a self-digestion resistance treatment such as reductive dimethylation is also not performed, and thus, heat resistance is poor, and a pH tolerance range, a compatible buffer solution and pH thereof are also limited.

A stock solution was prepared by dissolving a protease substrate in DMSO such that a final concentration is 10 mM. A substrate solution, a reaction buffer solution, a non-immobilized (free) protease solution or an immobilized protease suspension were mixed at ratios shown in Table 1 to prepare an enzyme reaction solution.

**[Table 1]**

| Enzyme reaction solution composition | Content |
|---|---|
| Substrate solution | 10 µL (100 nmol) |
| Reaction buffer solution (25 mM Tris) | 500 µL |
| Immobilized protease suspension (0.5 mg/mL) | 25 µL |
| Non-immobilized protease suspension (0.5 mg/mL) | 5 µL |

An enzymatic reaction was performed by using the prepared enzyme reaction solution and by setting the following conditions. Additives of (c) were added to a reaction buffer solution (25 mM Tris) such that a predetermined final concentration was obtained.

(a) Temperature
   25 °C, 37 °C, 45 °C, 50 °C, 60 °C, 70 °C
(b) pH
   6.5, 7.0, 7.5, 8.0, 8.5, 9.0
(c) Additives (a protein denaturing agent, a surfactant, an organic solvent and the like / temperature: 37 °C; pH: 8.0)
   1M, 2M urea
   0.1% n-octyl-β-D-thioglucoside (OTG)
   10%, 20%, 50% acetonitrile (MeCN)
   5 mM, 10 mM, 20 mM dithiothreitol (DTT)
   1 mM, 5 mM, 10 mM tris (2-carboxyethyl) phosphine hydrochloride (TCEP)
   0.1% CHAPS
   0.1% SDS
   0.1% Tween 20
   0.1% Triton X-100
   0.1% NP-40
   50 mM, 150 mM, 500 mM NaCl
   50 mM, 150 mM, 500 mM AS
   50 mM IAA
   50 mM, 500 mM trehalose
   10% glycerol
   10 mM EDTA

The enzymatic reaction was performed under the respective conditions for 1.5 hours while vortex stirring was performed. At the end of the reaction, 50 µL of 2N-HCl or a 10% sulfuric acid was added to completely stop the enzymatic reaction. After the nanoparticles were removed by filtration with a multi-screen filter plate, the solution was dispensed into an optical bottom plate, absorbance (405 nm, extinction coefficient = 9920 M⁻¹·cm⁻¹) of paranitroaniline (p-NA) released from the substrate was measured using a microplate reader (TECAN Infinite M200 Pro), and enzyme activity was evaluated.

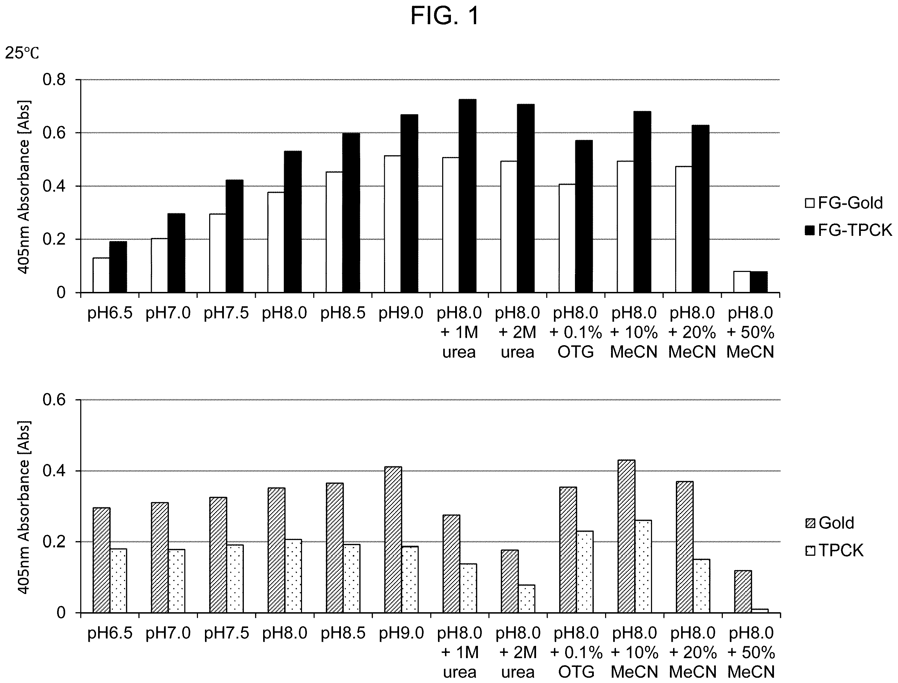

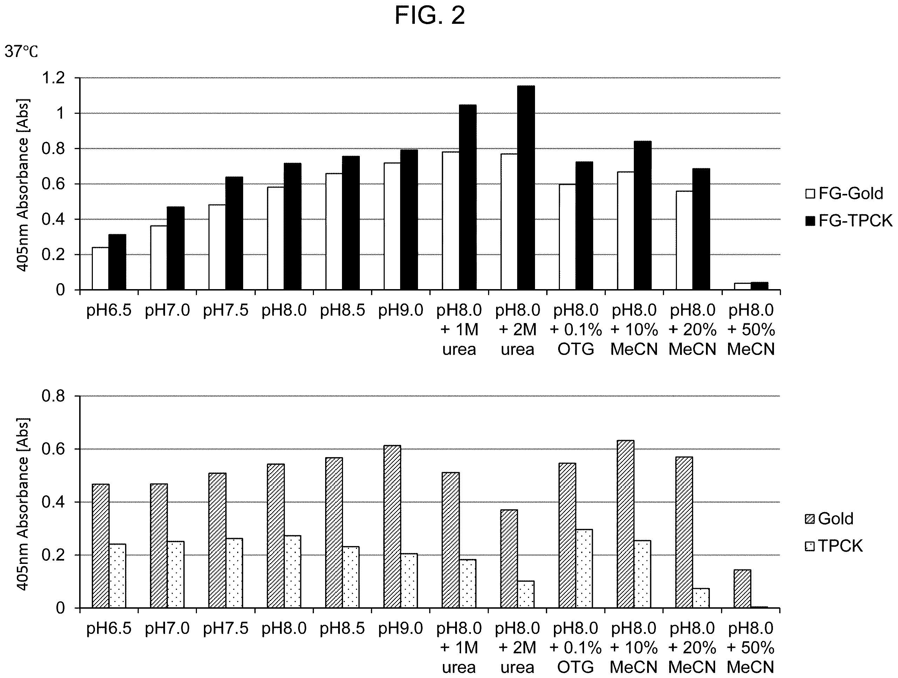

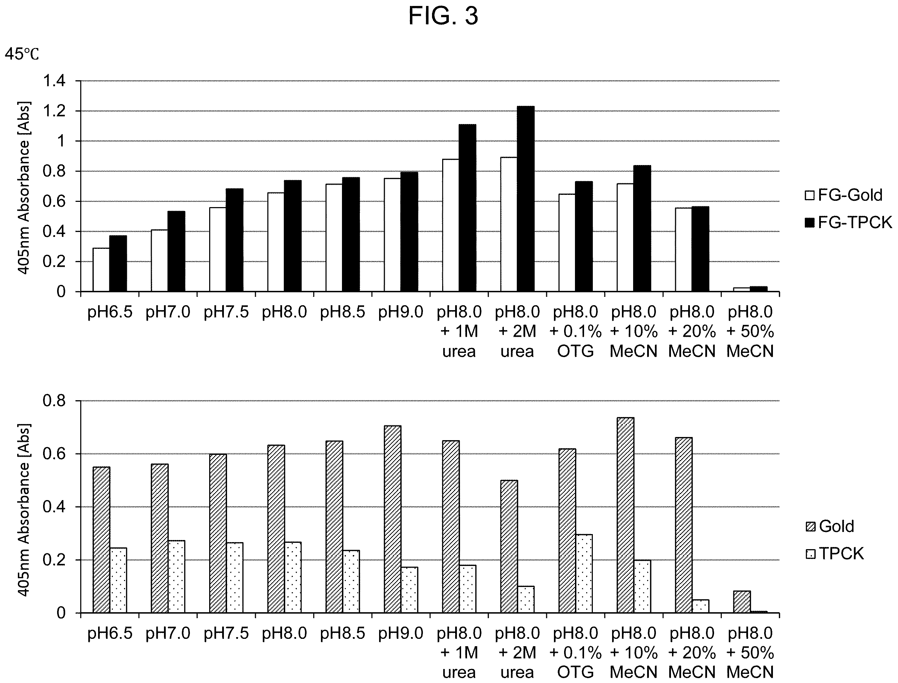

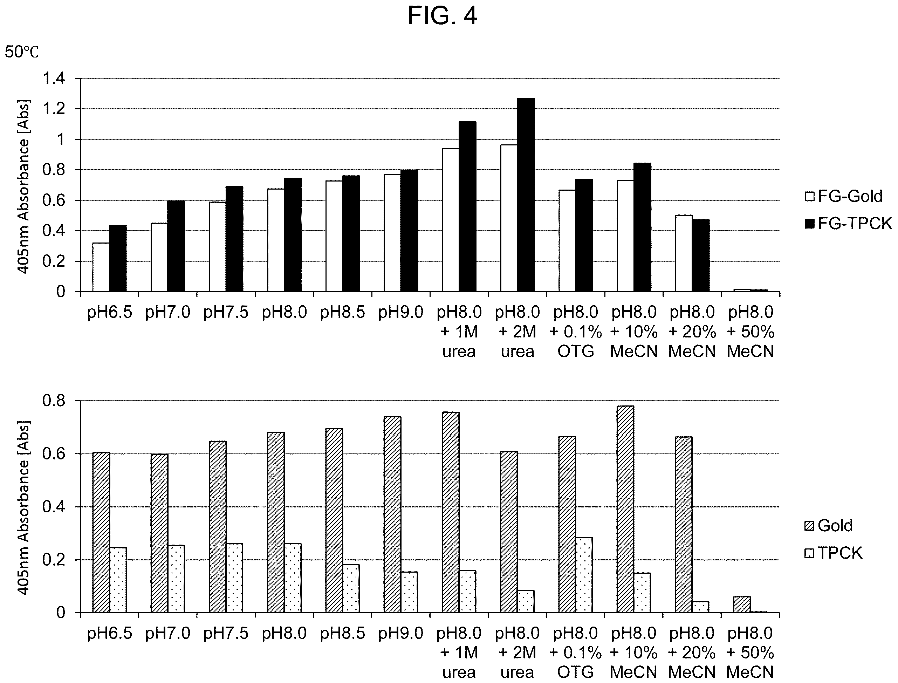

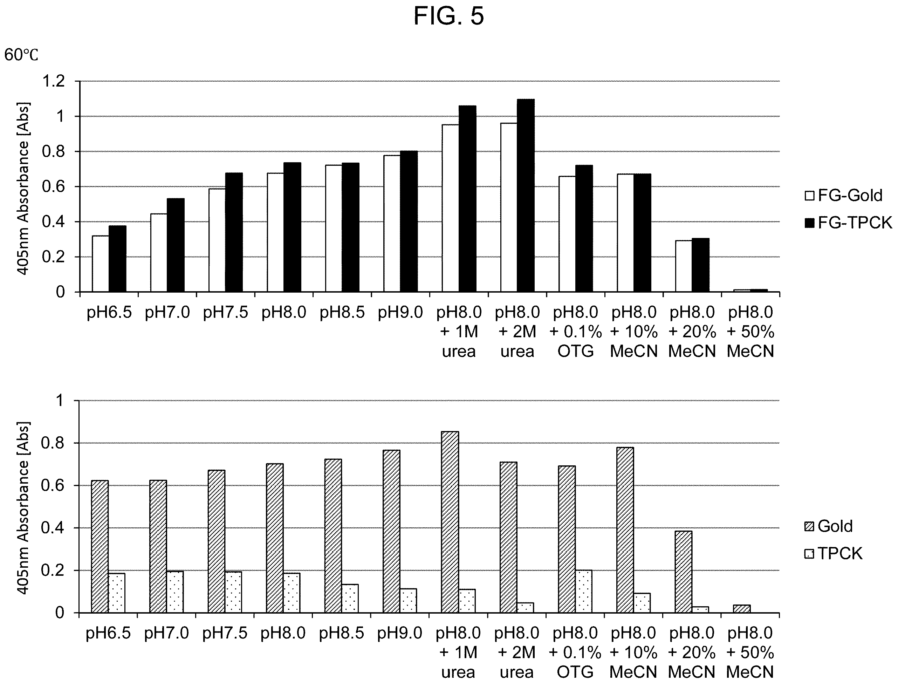

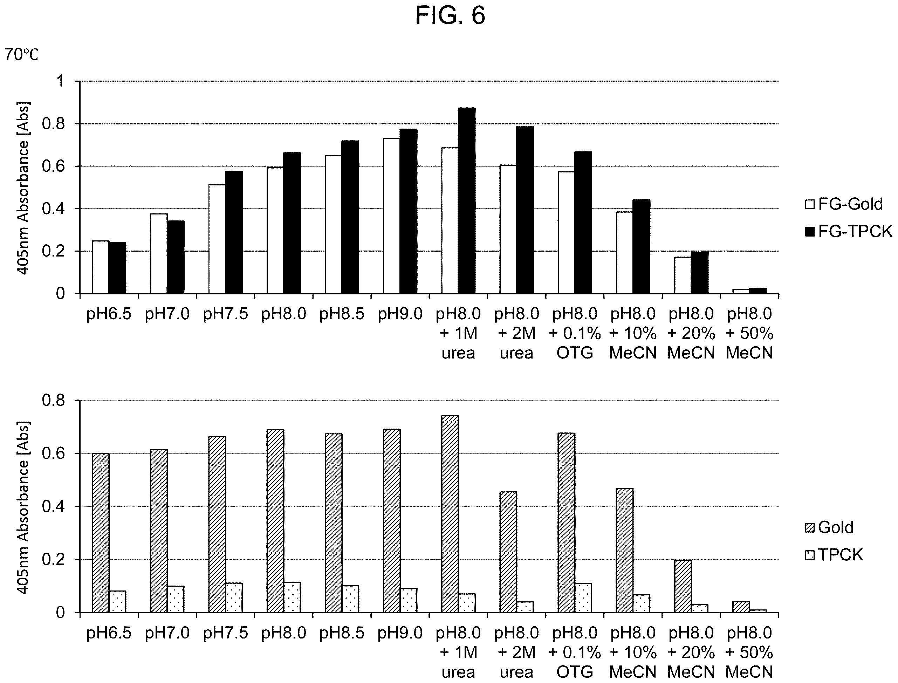

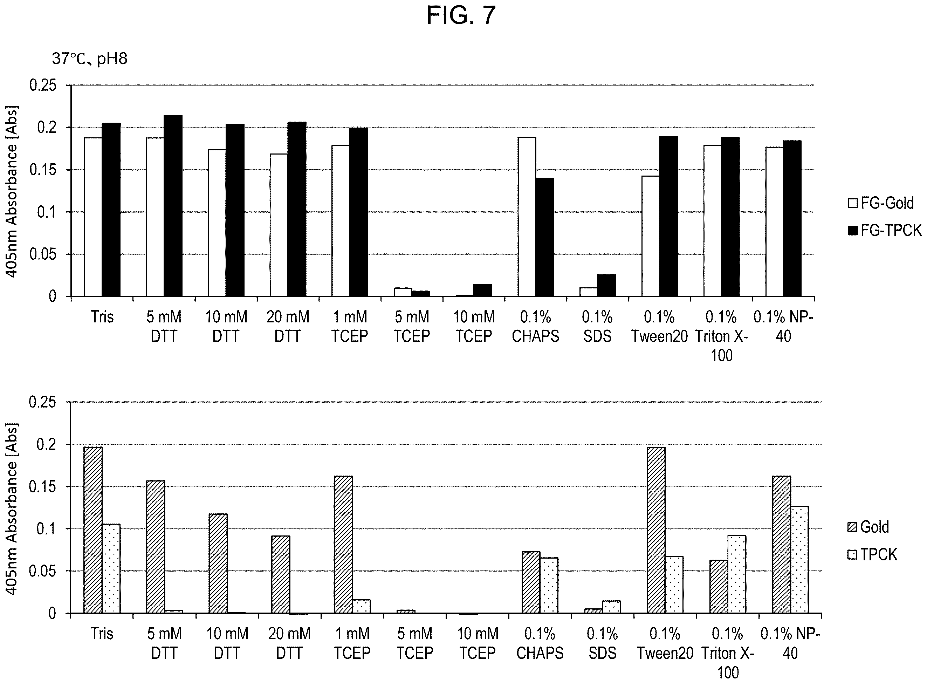

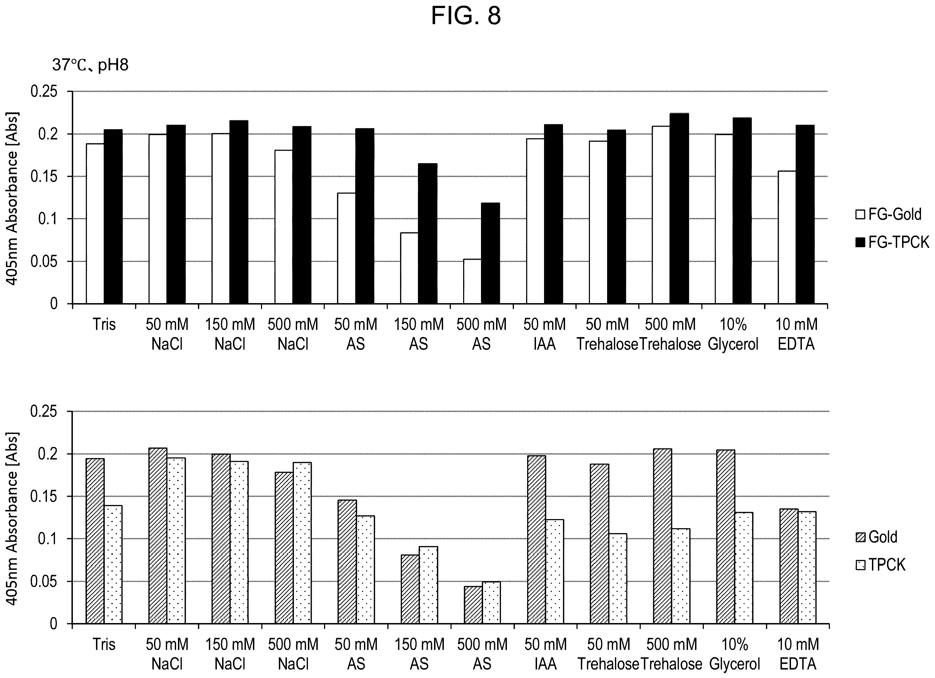

The results are shown in Figs. 1 - 8. Gold has trypsin activity in a very wide temperature range of 25 - 70 °C regardless of pH and additives, whereas trypsin activity of TPCK was weak, especially remarkably lower at temperatures of 60 °C or higher (see the lower panels of Figs. 1 - 6). In contrast, trypsin activity of TPCK (FG-TPCK) immobilized on FG beads is dramatically increased, regardless of temperature and pH, and exceeds or about the same as that of Gold (FG-Gold) immobilized on FG beads (the upper panels of Figs. 1 - 6). In particular, in the presence of urea, which is a protein denaturing agent often used in proteomics, TPCK is in a state equivalent to having lost trypsin activity despite being at an optimum pH (pH 8) for trypsin, whereas FG-TPCK retained its activity exceeding that of FG-Gold (see the upper panels of Figs. 1 - 6). Even under an environment of other additives (a protein denaturing agent, a surfactant, an organic solvent, and the like), the trypsin activity of FG-TPCK remarkably increased as compared to TPCK, and FG-TPCK and FG-Gold behaved substantially identically (see Figs. 7 and 8).

### (Example 3) Enzyme Stability Test 2 (Comparison between Nanoparticle-Immobilized Protease and Ordinary Particle-Immobilized Protease)

"FG-Gold" and "FG-TPCK" were respectively prepared by using FG beads (FG beads NHS manufactured by Tamagawa Seiki Co., Ltd.) as nanoparticles and by immobilizing "Gold" and "TPCK" in the same way as in Example 1, and were each suspended in a Tris buffer (100 µL) (protease concentration in the suspension: 0.5 µg/µL). Commercially available Promega Immobilized Trypsin (Cellulose resin) (hereinafter referred to as "CR-TPCK") or Pierce Immobilized TPCK Trypsin (4% crosslinked Agarose resin) (hereinafter referred to as "AR-TPCK") was used as ordinary particle (microparticle)-immobilized protease. The particles were washed five times with 25 mM Tris at pH 8.0 and then made into a slurry of 75 ml.

A stock solution was prepared by dissolving a protease substrate (N-α-benzoyl-DL-arginine-p-nitroanilide hydrochloride) in DMSO such that a final concentration is 10 mM. An enzyme reaction solution was prepared by adding 50 µL of a substrate solution, 25 µL of a nanoparticle-immobilized protease suspension or 12.5 µL of an ordinary particle (microparticle)-immobilized protease suspension to 500 µL of a reaction buffer solution (25 mM Tris).

An enzymatic reaction was performed by using the prepared enzyme reaction solution and by setting the following conditions. Additives of (b) were added to a reaction buffer solution (25 mM Tris) such that a predetermined final concentration was obtained.

(a) pH (Temperature :37 °C)
   6.5, 7.0, 7.5, 8.0, 8.5, 9.0
(b) Additives (protein denaturing agent and the like / pH 8.0; temperature 37 °C)
   1M, 2M urea
   50 mM, 150 mM, 500 mM NaCl
   50 mM, 150 mM, 500 mM AS
   50 mM IAA
   10 mM EDTA
   50 mM, 500 mM trehalose
   10% glycerol

The enzymatic reaction was performed under the respective conditions for 1.5 hours while vortex stirring was performed. At the end of the reaction, 50 µL of a 10% sulfuric acid was added to completely stop the enzymatic reaction. After the nanoparticles were removed by filtration with a multi-screen filter plate, the solution was dispensed into an optical bottom plate, absorbance (405 nm, extinction coefficient = 9920 M⁻¹·cm⁻¹) of paranitroaniline (p-NA) released from the substrate was measured using a microplate reader (TECAN Infinite M200 Pro), and enzyme activity was evaluated. Evaluation of enzyme activities of the commercial available products was performed by comparing relative enzyme activities with enzyme activity at pH 8.0 as 1.

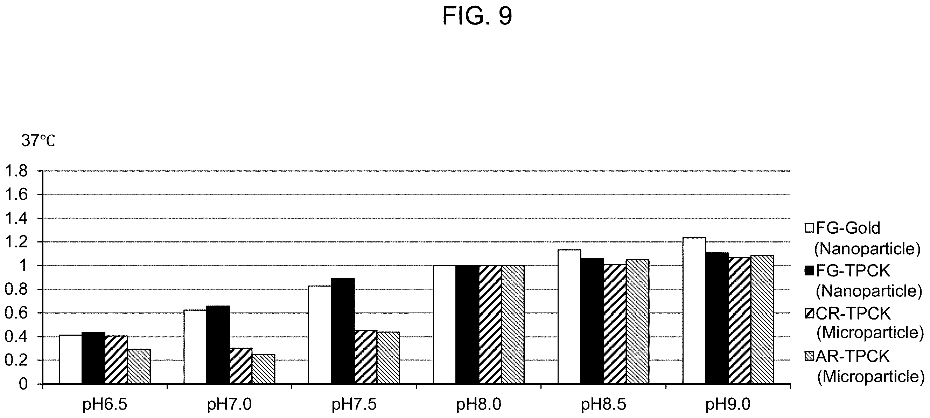

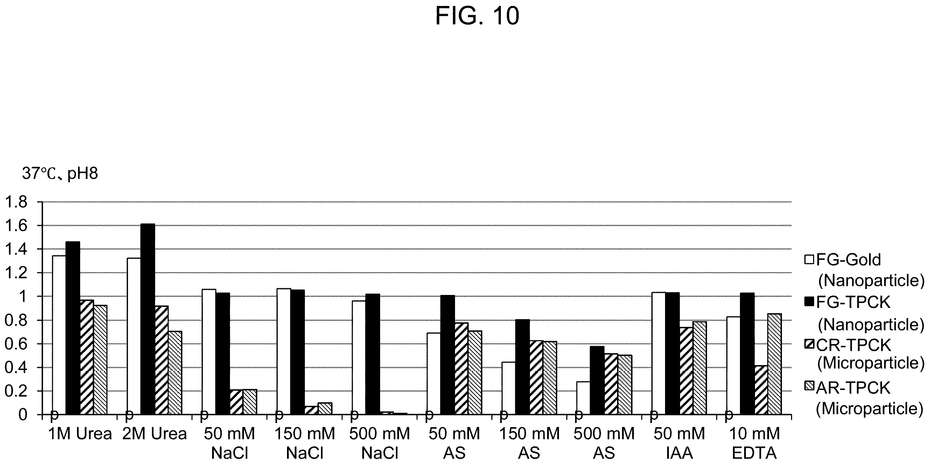

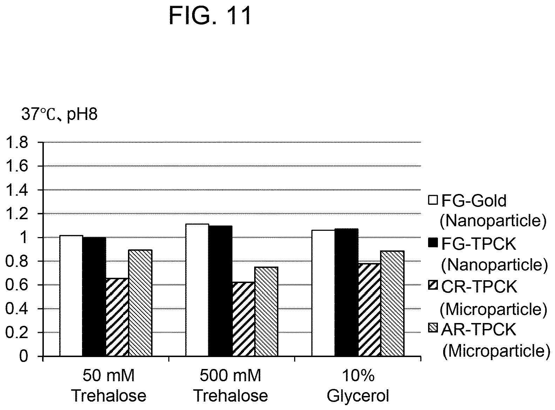

The results are shown in Figs. 9 - 11. The nanoparticle-immobilized proteases have higher enzyme activities than the microparticle-immobilized proteases in the vicinity of neutrality (pH 7.0 - 7.5), and are able to maintain the activities over a wide pH range including the alkaline side (Fig. 9). From this result, a nanoparticle immobilized protease is advantageous, for example, in a case where a sample such as a human body fluid or blood of about pH 7 is digested. Further, in an environment of a protein denaturing agent (urea), a salt (NaCl), a salting-out agent (AS), a protein reducing and alkylating agent (IAA), or a protein protecting and stabilizing agent (EDTA, Trehalose, or Glycerol), the nanoparticle-immobilized proteases had higher enzyme activities than the microparticle-immobilized proteases (Figs. 10 and 11). In particular, it is fatal that the microparticle-immobilized proteases have no resistance to NaCl, whereas the nanoparticle-immobilized proteases were stable even in the presence of NaCl at high concentrations. A tendency was observed that, for the microparticle-immobilized proteases, when a stabilizer is present, the enzyme activity rather decreases. In contrast, in the nanoparticle-immobilized proteases, a reason why the enzyme activity does not decrease even when a stabilizer is present is that the particles are nano-sized, and thus, a decrease in dispersibility (probability of being in contact with the substrate) associated with an increase in viscosity of the solution is unlikely to occur. From the above, it can be said that a nanoparticle-immobilized protease is suitable for application to a clinical examination test or the like in which a protein or the like in a crudely purified biological sample is to be analyzed.

### [Industrial Applicability]

The present invention can be used in a reagent manufacturing field for evaluation and analysis tests of products in the development of biopharmaceuticals such as antibody drugs and protein drug products, and for clinical examination at clinical sites.

All publications, patents and patent applications cited in the present specification are incorporated by reference in their entirety in the present specification.

## Claims

1. An immobilized protease obtained by immobilizing a crudely purified protease or a protease that has not been subjected to a self-digestion resistance treatment on surfaces of nanoparticles.

2. The immobilized protease according to claim 1, wherein the nanoparticle has a particle size of 100 - 500 nm.

3. The immobilized protease according to any one of claims 1 and 2, wherein the nanoparticle is a magnetic nanoparticle.

4. The immobilized protease according to any one of claims 1 - 3, wherein the protease is trypsin, chymotrypsin, lysyl endopeptidase, V8 protease, Asp N protease, Arg C protease, papain, pepsin, or dipeptidyl peptidase.

5. The immobilized protease according to any one of claims 1 - 3, wherein the self-digestion resistance treatment is a reductive dimethylation treatment.

6. The immobilized protease according to claim 5, wherein the protease is trypsin or lysyl endopeptidase.

7. A method for preparing an immobilized protease comprising a process of immobilizing a crudely purified protease or a protease that has not been subjected to a self-digestion resistance treatment on surfaces of nanoparticles.

8. A method of imparting, to a crudely purified protease or a protease that has not been subjected to a self-digestion resistance treatment, resistance to a change in an external environment by immobilizing the protease on surfaces of nanoparticles.

9. The method according to any one of claims 7 and 8, wherein the protease is trypsin, chymotrypsin, lysyl endopeptidase, V8 protease, Asp N protease, Arg C protease, papain, pepsin, or dipeptidyl peptidase.

10. The method according to any one of claims 7 and 8, wherein the self-digestion resistance treatment is a reductive dimethylation treatment.

11. The method according to claim 10, wherein the protease is trypsin or lysyl endopeptidase.
